# EUROPEAN PATENT APPLICATION

(11) **EP 4 810 043 A1**
(43) Date of publication of application: **23.09.2026**
(21) Application number: 25165218.6
(22) Date of filing: 21.03.2025
(51) Int. Cl.: A61K 31/513, A61K 31/519, A61K 45/06, A61P 35/00

(54) **SHORT INFUSION [6R]-MTHF IN 5-FU BASED CANCER CHEMOTHERAPY**

(71) Applicant: Isofol Medical AB, 413 46 Göteborg (SE)
(72) Inventor: Tell, Roger, 416 46 Gothenburg (SE)
(74) Representative: AWA Denmark A/S

(57) **Abstract**

The present invention relates to the treatment of gastrointestinal cancer, incl. metastatic colorectal cancer (mCRC) in human populations, employing a dosage regimen which includes a short infusion of arfolitixorin, *i.e*. [6R]-5,10-methylenetetrahydrofolate ([6R]-MTHF) in connection with 5-fluorouracil (5-FU) based chemotherapy.

## Description

### BACKGROUND OF THE INVENTION

In 2020, more than 1.9 million new cases of colorectal cancer (CRC) and more than 930,000 deaths due to CRC were estimated to occur globally, representing about one in 10 cancer cases and deaths. Colon and rectal cancer represented 6.0% and 3.8%, respectively, of the new cancer cases and 5.8% and 3.4% of new deaths. Overall, CRC ranks third in terms of incidence, but second in terms of mortality (Sung et al, 2021). In 2022, an estimated 106,180 new cases of colon cancer and 44,850 cases of rectal cancer were estimated to occur in the US, and an estimated 52,580 people were to die of colon and rectal cancer combined (Siegel et al, 2022).

Despite improvements in screening methodologies, approximately 20% of patients are diagnosed with metastatic colorectal cancer (mCRC), which carries a 14% 5-year survival rate. Treatment of mCRC is for the most part palliative in nature. For patients with unresec-table mCRC (representing more than 80% of this population), median survival has significantly increased up to 30 months due to advances in multi-modal management, modern cytotoxic chemotherapy (CTC) and the introduction of targeted agents (Aparicio et al, 2020; ASCO Cancer.Net. March 2024) and immunotherapy with checkpoint inhibitors.

The treatment of CRC is based on e.g. patient age and stage of the disease. Surgery has a major role in the treatment of early- to mid-stages (Stage I-III). About 95% of advanced or metastatic (Stage IV) CRC patients are treated with systemic chemotherapy, and only about 5% are eligible for immunotherapy/checkpoint inhibitors. Post-operative adjuvant chemotherapy with cytotoxic agents is recommended as standard clinical practice for patients with high-risk Stage III disease *i.e.,* adjuvant treatment (Engstrom et al, 2009). The management of Stage IV mCRC, *i.e.,* palliative treatment, requires the systemic administration of cytotoxic drugs (Santi et al, 1974) or targeted with checkpoint inhibitors for specific subgroups of patients.

The established current standard-of-care (SoC) systemic therapy for mCRC is based on 5-fluorouracil (5-FU) biomodulated with folinic acid (i.e., LV or L-LV) in diverse combinatory chemotherapy regimens including oxaliplatin or irinotecan as part of the FOLFOX or FOLFIRI regimens, often in combination with a biologic drug (Cervantes et al, 2023; NCCN Guidelines Colon Cancer v. 1.2024). Similarly, FOLFIRINOX is the SoC combination for mPADC (NCCN Guidelines Version 2.2023; Conroy et al, 2023) and also for mCRC in relevant population (e.g. younger patients with a need for a quick response due to symtoms).

Treatment results have improved over time but the response rate even after addition of folates and oxaliplatin remains below 50% (Gustavsson et al, 2015) or slightly higher. Supplementation with additional cytostatic and immunological agents has brought only modest improvements for most patients. The therapeutic need thus remains high in gastrointestinal cancer, in particular mCRC.

### DEFINITIONS

As used herein, the term **Leucovorin^{®}** or **folinic acid** shall both mean 5-formyl tetrahydrofolic acid, i.e. the 5-formyl derivative of tetrahydrofolic acid. Folinic acid contains 2 asymmetric centers. **Leucovorin^{®}** (LV) is composed of a 1:1 mixture of the two dextrorotary and levorotary diastereomers (*d*-leucovorin (*d*-LV, (6R,2'S)-configuration) and *l*-leucovorin (*l*-LV, (6S,2'S)-configuration), respectively), and may also be referred to as **(d,*l*-LV).**

As used herein, the term **Levoleucovorin** shall refer to the commercially available product which contains only the natural and pharmacologically active *levo*-isomer *l*-LV (or LLV). In vitro, *l*-LV has been shown to be rapidly converted to the biologically available methyl-tetrahydrofolate form while the *dextro* form *d*-LV (DLV) is slowly excreted by the kidneys. Leucovorin and levoleucovorin have however been shown to be pharmacokinetically identical and may be used interchangeably with limited differences in efficacy (considering the inactive d-isomer in LV) or side effects (see Kovoor et al, Clin Colorectal Cancer 8 200-6 (2009).

As used herein, the terms **MTHF, CH2-THF, or methyleneTHF** shall all refer to 5,10-methylene-5,6,7,8-tetrahydrofolate. As used herein, the terms **racemic methyleneTHF, CoFactor^{®} or [6R,6S]-5,10-methyleneTHF** shall all refer to the 1:1 diastereomeric mixture [6R,6S]-5,10-Methylene-5,6,7,8-tetrahydrofolate.

As used herein, the terms **arfolitixorin** and [6R]-5,10-MTHF shall both refer to the single diastereomer, [6R]-5,10-methylenetetrahydrofolate. As used herein, the terms IV or i.v. shall both mean **intravenous.**

As used herein, the term **ORR** shall refer to the Objective Response Rate, i.e. the proportion of patients with a reduction in tumor burden of a predefined amount. This shall be calculated as follows: ORR = Sum of partial responses plus complete responses as per RECIST 1.1 (a set of published rules (link: https://recist.eortc.org/recist-1-1-2/) that define when tumors in cancer patients progress during treatments, the responses being defined as:
**Complete Response (CR):**
   - Disappearance of all target lesions. Any pathological lymph nodes (whether target or non-target) must have reduction in short axis to <10 mm.
**Partial Response (PR):**
   - At least a 30% decrease in the sum of diameters of target lesions, taking as reference the baseline sum diameters.
**Progressive Disease (PD):**
   - At least a 20% increase in the sum of diameters of target lesions, taking as reference the smallest sum on study (this includes the baseline sum if that is the smallest on study).
   - In addition to the relative increase of 20%, the sum must also demonstrate an absolute increase of at least 5 mm. (Note: the appearance of one or more new lesions is also considered progression).
**Stable Disease (SD):**
   - Neither sufficient shrinkage to qualify for PR nor sufficient increase to qualify for PD, taking as reference the smallest sum diameters while on study.

As used herein, the term **BSA** refers to Body Surface Area

As used herein, the term **CRC** refers to colorectal cancer. The term **mCRC** shall refer to metastatic colorectal cancer.

**FOLFIRI** is a chemotherapy regimen consisting of leucovorin, fluorouracil, and irinotecan.

**FOLFOX** is a chemotherapy regimen consisting of leucovorin, fluorouracil, and oxaliplatin.

**FOLFOXIRI** and **FOLFIRINOX** are chemotherapy regimens consisting of leucovorin, fluorouracil, irinotecan, and oxaliplatin.

### SUMMARY OF INVENTION

Arfolitixorin (the hemisulfate salt of [6R]-MTHF) is a novel folate-based compound in development for use in combination chemotherapy with 5-fluorouracil (5-FU) in the treatment of adult patients with cancers for which the combination of folates with 5-FU is part of the basic chemotherapeutic backbone such as mCRC. To increase the response rate of 5-FU in tumor treatment, folates are used in combination with 5-FU (Thirion et al, 2004; Machover et al, 1982; Machover et al, 1986; Piedbois et al, 1992; Longley et al, 2003) to biomodulate the activity of 5-FU in diverse combinatory chemotherapy regimens. All current folate-based therapies used in cancer treatment are prodrugs that need multiple activation steps into an active metabolite (5,10-methylenetetrahydrofolate [MTHF]) to exert their actions.

Arfolitixorin is the key active metabolite of both leucovorin (LV; [6RS]-5-formyl-5,6,7,8-tetrahydropteroyl-L-glutamic acid calcium salt) and its pure enantiomer L-leucovorin (L-LV), and was created as a product that does not require metabolic/genetically regulated activation to exert its action as a biomodulator of 5-FU activity via the enzyme thymidylate synthase (TS). Arfolitixorin is thus the first and only immediately active folate that potentiates 5-FU cytotoxicity.

It was therefore hypothesized that arfolitixorin might be superior compared to LV in the mFOLFOX regimen for metastatic CRC, which was evaluated in the ISO-CC-007 study (Taberenero et al, 2024). The ISO-CC-007 study was a randomized, phase III study in which patients with mCRC were randomized to receive arfolitixorin (120 mg/m² given as two intravenous bolus doses of 60 mg/m² 30 and 60 minutes after the 5-FU bolus, respectively) or LV (400 mg/m2 given as a single intravenous infusion before the 5-FU bolus) plus 5-FU, oxaliplatin, and bevacizumab.

Surprisingly, the ISO-CC-007 study did not show any statistically significant difference between arfolitixorin and LV treatment in the primary endpoint, which was overall response rate (ORR). As arfolitixorin was given as a split intravenous dose instead of a single dose, and in addition at a lower dose than LV, it was initially thought that the arfolitixorin arm of the study may have had inadequate tumor tissue levels of MTHF, leading to inferior TS inhibition.

However, a post-hoc per protocol (PP) analysis of the ISO-CC-007 study showed that arfolitixorin actually was superior to leucovorin with statistical significance in certain key regions, and numerically better across all trial sites, but only for patients with high compliance to the study protocol regarding the following two aspects where adherence was low:
- duration of the 5-FU bolus, and
- time interval between the 5-FU bolus and arfolitixorin.

Thus, adherence to the timing between the first and second arfolitixorin dose (30-60min) was high, but as regards the duration of the 5-FU bolus, non-compliance, defined by <80% doses given within the 2-4 min PP window, was reported for 101 (42%) in the ARF and 97 (41%) in the LV arm, respectively. Further, for the length of time between the 5-FU bolus and first bolus arfolitixorin, non-compliance, defined by <80% doses given within the 25-35 min PP window, was reported for 92 (38%) in arfolitixorin patients.

Applicant reasoned that this implies:
- The post-hoc per protocol analysis indicates superior efficacy of arfolitixorin in the ISO-CC-007 dosing regimen provided 5-FU and arfolitixorin are administered with precision, and
- High accuracy regarding the timing and duration of administration is important.

As these prerequisites may be complicated to comply with in clinical practice, applicant realized there remains a need for a more robust treatment regimen comprising arfolitixorin and 5-FU which is less prone to underperformance caused by protocol non-compliance.

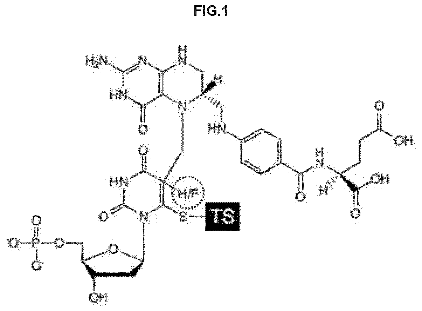

This has led applicant to continue the research into the underlying interaction between 5-FU, TS and CH2-THF (arfolitixorin), see Figures 1-3 herein, by both *in vitro* and *in vivo* studies, which has led to the surprising finding that by modifying the ISO-CC-007 regimen by:
- administering arfolitixorin as a single short infusion (10 ±3 min) instead of by two discrete rapid (<3 min) boluses, and
- administering 5-FU as a bolus 15 min after arfolitixorin instead of 30 + 60 min before, and
- introducing a 15-min time gap between the administration of oxaliplatin and 5-FU,
best ORRs (objective response rates) of >55%, such as >60% can be achieved.

Accordingly, in **a first aspect** of the invention, arfolitixorin is provided for use in a human patient in the treatment of gastrointestinal cancer, including metastatic colorectal cancer, which treatment comprises the following steps:
a. administering a continuous IV infusion containing bevacizumab (5 mg/kg), followed by
b. administering a continuous IV infusion containing 85 mg/m² oxaliplatin, concomitant with
c. administering a short IV infusion over 10 min ± 3 min containing 200 - 500 mg/m² arfolitixorin, followed, 15 ± 3 minutes after completing the short infusion, by
d. administering an IV bolus containing 400 mg/m² 5-fluorouracil (5-FU), followed immediately by
e. administering a continuous IV infusion containing 2400 mg/m² 5-FU over 46 hours, wherein step b) and step c) are completed concurrently, and wherein step d) is initiated 25 minutes ± 3 min after initiating step c).

In a **second aspect** of the invention there is provided a method of treating a human diagnosed with gastrointestinal cancer, including metastatic colorectal cancer, which method comprises the following steps:
a. administering a continuous IV infusion containing bevacizumab (5 mg/kg), followed by
b. administering a continuous IV infusion containing 85 mg/m² oxaliplatin, concomitant with
c. administering a short IV infusion over 10 min ± 3 min containing 200 - 500 mg/m² arfolitixorin, followed, 15 ± 3 minutes after completing the short infusion, by
d. administering an IV bolus containing 400 mg/m² 5-fluorouracil (5-FU), followed immediately by
e. administering a continuous IV infusion containing 2400 mg/m² 5-FU over 46 hours, wherein step b) and step c) are completed concurrently, and wherein step d) is initiated 25 minutes ± 3 min after initiating step c).

The treatment according to any of the aspects of the present invention will continue until disease progression (radiographic and/or clinical), unless unacceptable toxicity, death, patient withdrawal or general or specific changes in the patient's condition render the patient unacceptable for further treatment in the judgment of the Investigator.

The treatment steps a) - e) according to any of the aspects of the present invention are thus repeated every 2 weeks until progressive disease (PD), or clear clinical deterioration according to the Investigator's judgment, and as long as the patient is tolerating the treatment and agrees to continue.

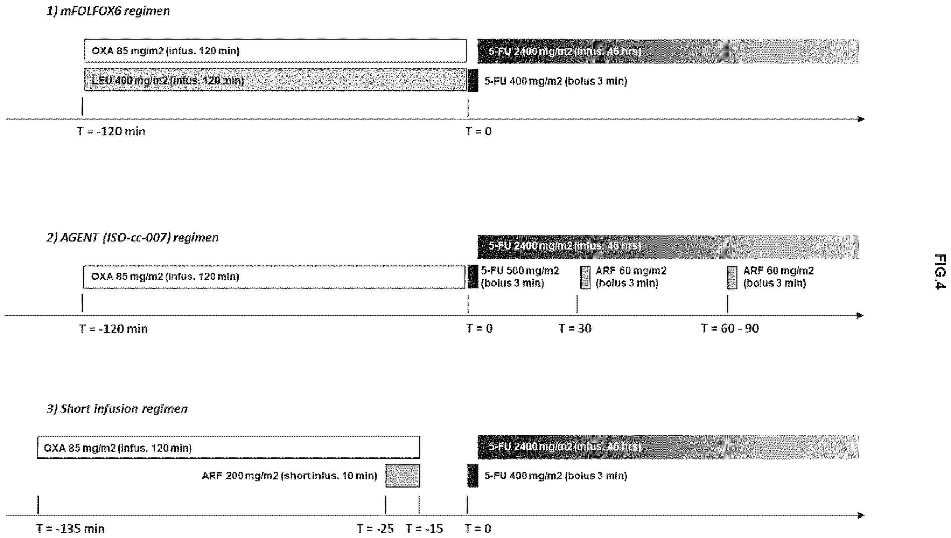

The three regimens discussed hereinabove (mFOLFOX, ISO-cc-007 and the regimen of the present invention) are illustrated in Figure 4 herein.

### DESCRIPTION OF FIGURES

**Fig. 1****.** Ternary complex formed between [6R]-5,10-methylene-THF, thymidylate synthase (TS) and either 2'-deoxy-uridine-5'-monophosphate (2-dUMP) or 5-fluoro-2'-deoxy-uridine-5'-monophosphate, (5-FdUMP). The dotted circle marks the difference between 2-dUMP and 5-FdUMP. **THF:** tetrahydrofolate; **TS:** thymidylate synthase. Adapted from Danenberg (2016).

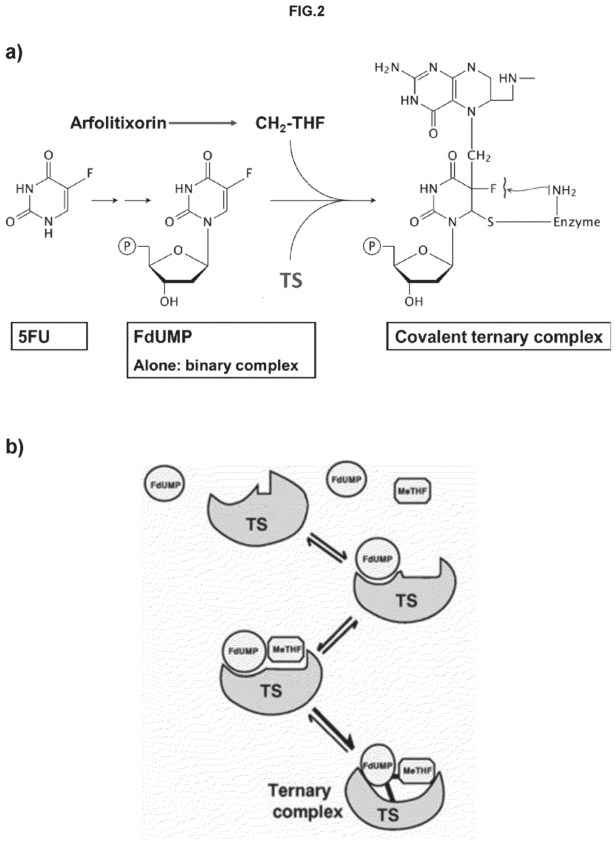

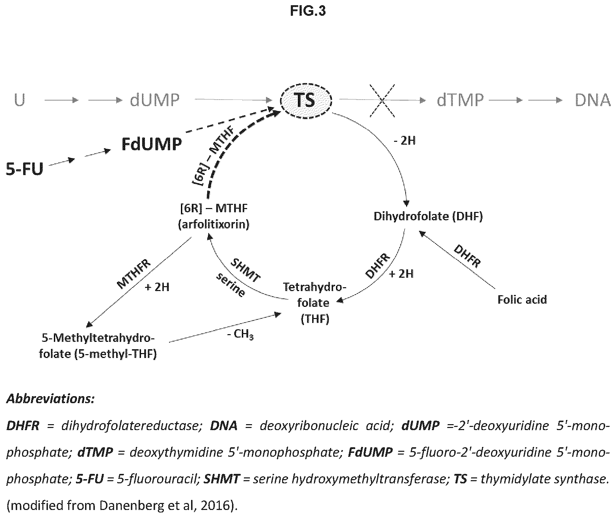

**Fig. 2****.** Mechanism of action and regulation of TS. 5-FU has to be converted to FdUMP via several reactions (see Figure 3), which can then form an unstable binary complex FdUMP-TS, but in the presence of CH2-THF, FdUMP forms a stable covalent ternary complex with TS (see part b of Figure 2). The concentration of CH2-THF is increased by administration of LV or arfolitixorin (A). Adapted from Peters (2020).
**Fig. 3****.** A schematic presentation of the arfolitixorin ([6R]-MTHF) metabolism and bio-chemical pathway in the presence of 5-FU. 5-FU is initially converted to the active metabolite FdUMP which causes cytotoxicity by inhibiting TS, the key enzyme involved in DNA replication and repair through the synthesis of deoxythymidine nucleotides (Parker and Cheng 1990; Longley et al, 2003). By inhibiting TS, 5-FU suppresses DNA synthesis in the cells leading to cell death by apoptosis. The inhibition is effected by the formation of a ternary complex between FdUMP, TS and CH2-THF and strengthened by an excess of arfolitixorin.
**Fig. 4****.** The figure shows the difference between the initial dosing of oxaliplatin (OXA), folate (*i.e*. leucovorin (LEU) or arfolitixorin (ARF)), and 5-fluorouracil (5-FU) in ¹⁾ the modified FOLFOX6 regimen, ²⁾ the AGENT (ISO-cc-007) study regimen, and ³⁾ the short infusion regimen of the present invention. The administration of bevacizumab (where relevant) is not shown. A bolus of 5-FU is administered at T = 0 on Day 1 for all three regimens, and the timing of the administration of the other two components is calculated from this time point.

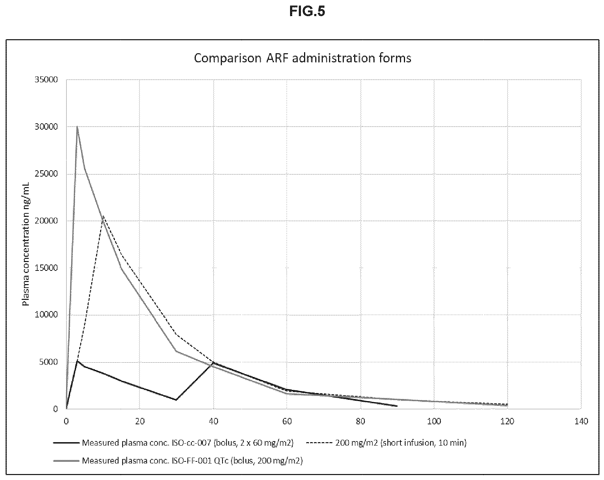

**Fig. 5****.** An overlay of the achieved exposure in ¹⁾ the ISO-CC-007 clinical study (black line), ²⁾ the ISO-FF-001 clinical study (dark grey line) together with ³⁾ the model-predicted (simulated) plasma concentration following an IV infusion of 200 mg/m² ARF over 10 minutes (stippled line).

### DETAILED DESCRIPTION OF THE INVENTION

As mentioned, arfolitixorin ([6R]-5,10-methylenetetrahydrofolate, [6R]-MTHF) is a novel folate which has been in development for a number of years and has been studied in several clinical studies together with 5-fluorouracil (5-FU).

The effect of 5-FU has long been known to be increased (response rate and overall survival) by concurrent (or pre-) treatment with leucovorin (LV). A racemic mixture of the natural (S) and unnatural (R) diastereoisomers of LV is most often administered, usually in the form of calcium folinate. LV is metabolized intracellularly to the active metabolite 5,10-methylenetetrahydrofolate (CH2-THF). Arfolitixorin is a stable form of CH2-THF, and thus needs no metabilization in contrast to the SoC folates LV and L-LV.

CH2-THF is the methyl-donor essential for the action of thymidylate synthase (TS), which catalyzes the reductive methylation of deoxyuridine monophosphate (dUMP) to deoxythymidine monophosphate (dTMP). dTMP is subsequently phosphorylated to deoxythymidine triphosphate (dTTP), which is required for DNA replication and repair.

After entering the cells, 5-FU is converted to 5-fluoro-2'-deoxyuridine 5'-monophosphate (FdUMP), which forms a stable, reversible ternary complex between TS, FdUMP, and CH2-THF (see Figure 1) leading to inhibition of the TS enzyme activity. Since the rate-limiting TS inhibition exploits one of the few metabolic bottlenecks in DNA synthesis, drugs inhibiting TS can be considered as first examples of targeted therapy.

The ternary complex between FdUMP, TS and CH2-THF is more stable than the complex formed with naturally occurring dUMP. This increased stability is due to several factors (Peters 1988, Gaurav 2020, Peters 1995):
1. FdUMP has a higher binding affinity for TS compared to dUMP. The Ki value for FdUMP binding to human ThyA (TS) is 1.7 nM, while dUMP has a Km of 2.5 µM.
2. FdUMP forms a covalent ternary complex with TS and CH2-THF, which is more stable than the non-covalent complex formed with dUMP.
3. The binding of FdUMP induces conformational changes in TS that further stabilizes the ternary complex.
4. The fluorine atom in FdUMP forms additional interactions within the TS binding pocket, contributing to the stability of the complex.

FdUMP alone forms a binary complex with TS which is not very stable, but in the presence of CH2-THF this is rapidly converted (reversably) to a ternary complex, see Figure 2, top. The presence of a high concentration of CH2-THF will shift the equilibrium towards the formation of the ternary complex, preventing the binary FdUMP-TS complex from being cleaved (Figure 2, bottom).

The FdUMP-TS complex formation and stability thus depends on the cellular concentration of both CH2-THF and FdUMP. In addition, the stabilizing effect of CH2-THF increases by the level of its polyglutamylation by attachment of up to five glutamate molecules which leads to a higher binding affinity towards TS (Gangjee 2006). Cancer cells further tend to have higher levels of folylpolyglutamate synthase (FPGS) activity which leads to increased polyglutamylation of folates, including CH2-THF.

Potentiation of 5-FU-induced TS inhibition by LV *(i.e.* indirectly by CH2-THF) has been demonstrated in many model systems, including cell lines and animal model systems for CRC, as well as in CRC patients. However, the LV-mediated increase of TS inhibition is limited both by the tissue distribution of LV and its subsequent cellular uptake and metabolism to CH2-THF. It has been hypothesized that direct administration of CH2-THF would bypass the limiting steps in metabolism, and arfolitixorin (the stable hemisulfate salt of CH2-MTHF) was thus designed to replace reduced folates such as LV as biomodulator.

The difference in a clinical setting in human patients between leucovorin and arfolitixorin has recently been analyzed by Taflin et al. in the Modelle-1 Phase 2 clinical study (TS-inhibition in Colorectal Liver Metastases Comparing Arfolitixorin and Calciumfolinate), the primary objective of which is to compare the TS-inhibition capacity of the combination arfolitixorin/5-FU in contrast to Calciumfolinate/5-FU in tumour, adjacent liver parenchyma and plasma in patients with liver metastases from colorectal cancer (Study Details | Modelle 001, TS-inhibition in Colorectal Liver Metastases Comparing Arfolitixorin and Calciumfolinate | ClinicalTrials.gov, see Taflin H et al. BJC Rep. 2024 Nov 20;2(1):89. doi: 10.1038/s44276-024-00111-4). The Modelle-1 results thus indicate that higher levels of CH2-THF in metastases can lead to greater TS inhibition, and thereby to increased effectiveness of 5-FU treatment.

The results from the Modelle-1 study demonstrated that both 30 and 120 mg/m² arfolitixorin resulted in higher concentrations of [6R]-5,10-methylene-THF in metastases, both before and after 5-FU treatment, as compared to 60 mg/m² Leucovorin.

Administration of 30 or 120 mg/m² arfolitixorin resulted in a higher ratio of poly/mono-glutamylated folates compared to 60 mg/m² Leucovorin after 5-FU administration (p = 0.045 and p = 0.0049, respectively). A dose-dependent TS inhibition was seen after administration of 30 mg/m² arfolitixorin (54.2% ± 189) vs 120 mg/m² arfolitixorin (83.8% ± 431) and both 30 and 120 mg/m² arfolitixorin seemed to give a better mean TS inhibition than 60 mg/m² Leucovorin (25.4% ± 301, p = 0.081 and p = 0.0026, respectively). Thus, significantly higher concentrations of [6R]-5,10-methylene-THF, ratio of poly-/mono-glutamylated CH2-THF and TS inhibition was found in colorectal liver metastases in patients receiving treatment with arfolitixorin compared to standard leucovorin therapy.

Further, applicant's previous *in vitro* studies with patient-derived colorectal cancer tumoroids (Example 3) indicated that the efficacy of arfolitixorin increases with increased concentrations and that a clear dose-response relationship exists beyond the levels that were achieved in the ISO-CC-007 study.

The PK and systemic exposure of arfolitixorin were next evaluated in a number of clinical studies using IV bolus doses from 30 to 500 mg/m². Over the dose range investigated, the systemic exposure was found to be linear and predictable. The elimination half-live is consistently < 1 hour and does not change with dose. No changes in pharmacokinetics (PK) were observed between different treatment cycles.

Study ISO-CC-002 showed that significant amounts of CH2-THF can be found in tumour tissue both after treatment with arfolitixorin or L-leucovorin when measured about 2 hours (median values) after the IV bolus dose of either arfolitixorin or L-leucovorin. The concentrations of CH2-THF were about 2-3 fold higher after dosing of arfolitixorin when compared with the same dose (in mg/m²) of L-leucovorin.

Study ISO-CC-005 showed that the PK of arfolitixorin appears to be linear over the dose range of 30-240 mg/m², and no differences could be seen on systemic exposure between different cycles. Arfolitixorin was safe and well tolerated over the dose range tested.

Thus, it was concluded that model-based PK simulations and predictions are expected to be quite accurate and possible to apply in order to predict plasma concentrations of CH2-THF after various dose regimens.

Available safety data from applicant's previous clinical studies and pharmacokinetic (PK) simulations show that it is possible to administer significantly higher doses of arfolitixorin than the 2x60 mg/m² given in the ISO-CC-007 study.

In a healthy volunteer study (ISO-FF-001) to investigate QT effects, 33 subjects were randomized to placebo (n=9) and three doses of arfolitixorin, given as a 2-minute bolus injection in the following doses: 200 mg/m² (n=8); 350 mg/m² (n=8); or 500 mg/m2 (n=8). The study data indicate that in relation to baseline, arfolitixorin causes a dose-dependent increase in QTcF that is transient.

Population PK modelling based PK simulations were next performed (Example 1) to explore different IV infusion dose regimens. By varying both dose and infusion length a number of different Cmax and AUC values can be achieved. As an example, Figure 5 is an overlay of the achieved exposure in ISO-CC-007 and ISO-FF-001 together with the model-predicted (simulated) plasma concentration following a short IV infusion of 200 mg/m² over 10 minutes.

As can be seen from Figure 5, by administering 200 mg/m² arfolitixorin as a short infusion (10 ±3 minutes, stippled line) rather than as a rapid bolus (< 3 min, gray line), a high total exposure (measured by the AUC) is achieved whilst keeping a reasonable safety margin to peak plasma concentrations that are associated with a risk for QTc interval prolongation. As expected, the exposure achieved by the ISO-cc-007 regimen is much lower.

As mentioned hereinabove, the results of the ISO-cc-007 study - in which ARF did not demonstrate superiority vs. LV - have made applicant realize that there remains a need for a more robust treatment regimen comprising arfolitixorin and 5-FU which is less prone to underperformance caused by protocol non-compliance.

Applicant has concluded that this goal ultimately depends on stabilizing the ternary complex between FdUMP, TS and CH2-THF in tumor cells as much as possible. This again, theoretically, hinges on optimizing the binding affinity between CH2-THF and the initially formed binary complex between 5-FU and TS. Based on the results of the Modelle-1 study and PK simulations discussed above, applicant has reasoned that this may be achieved ¹⁾ by introducing a sufficient interval between administration of arfolitixorin and 5-FU, as this will allow arfolitixorin not only to be taken up by the tumor, but also being polyglutamylated which - due to higher binding affinity - allows for a more potent FdUMP mediated inhibition of TS and ²⁾ by increasing the dosing of arfolitixorin beyond the level employed in the ISO-cc-007 study in order to raise the amount of CH2-THF available for glutamylation, and finally ³⁾ by administering arfolitixorin by a short infusion rather than by bolus, as PK simulations predict this will allow administering higher doses (and thereby achieve a higher total exposure) without reaching peak plasma concentrations that may be associated with a risk for QTc interval prolongation.

This has led applicant to continue the research into the underlying interaction between 5-FU, TS and CH2-THF (arfolitixorin), see Figures 1-3 herein, by both *in vitro* and *in vivo* studies, which has led to the surprising finding that by modifying the ISO-CC-007 regimen by:
- administering arfolitixorin as a single short infusion (10 ±3 min) instead of by two discrete rapid (<3 min) boluses, and
- administering 5-FU as a bolus 15 min after arfolitixorin instead of 30 + 60 min before, and
- introducing a 15-min time gap between the administration of oxaliplatin and 5-FU,
best ORRs (objective response rates) of >55%, such as >60% can be achieved.

Accordingly, in a **first aspect** of the invention, arfolitixorin is provided for use in a human patient in the treatment of gastrointestinal cancer, including metastatic colorectal cancer, which treatment comprises the following steps:
a. administering a continuous IV infusion containing bevacizumab (5 mg/kg), followed by
b. administering a continuous IV infusion containing 85 mg/m² oxaliplatin, concomitant with
c. administering a short IV infusion over 10 min ± 3 min containing 200 - 500 mg/m² arfolitixorin, followed, 15 ± 3 minutes after completing the short infusion, by
d. administering an IV bolus containing 400 mg/m² 5-fluorouracil (5-FU), followed immediately by
e. administering a continuous IV infusion containing 2400 mg/m² 5-FU over 46 hours,
wherein step b) and step c) are completed concurrently, and wherein step d) is initiated 25 minutes ± 3 min after initiating step c).

In a **second aspect** of the invention there is provided a method of treating a human diagnosed with gastrointestinal cancer, including metastatic colorectal cancer, which method comprises the following steps:
a. administering a continuous IV infusion containing bevacizumab (5 mg/kg), followed by
b. administering a continuous IV infusion containing 85 mg/m² oxaliplatin, concomitant with
c. administering a short IV infusion over 10 min ± 3 min containing 200 - 500 mg/m² arfolitixorin, followed, 15 ± 3 minutes after completing the short infusion, by
d. administering an IV bolus containing 400 mg/m² 5-fluorouracil (5-FU), followed immediately by
e. administering a continuous IV infusion containing 2400 mg/m² 5-FU over 46 hours,
wherein step b) and step c) are completed concurrently, and wherein step d) is initiated 25 minutes ± 3 min after initiating step c).

In an embodiment of the first or second aspect, bevacizumab is administered over 100 min ± 5 min as a continuous IV infusion. In an embodiment of the first or second aspect, oxaliplatin is administered over 120 min ± 5 min as a continuous IV infusion. In an embodiment of the first or second aspect 5-fluorouracil (5-FU) is administered as an IV bolus over 2-4 minutes.

In another embodiment of the first or second aspect arfolitixorin is administered as a short IV infusion over 10 min ± 3 min. In an embodiment of the first or second aspect arfolitixorin is administered as a short IV infusion starting 15 minutes ± 2 minutes before the 5-FU rapid bolus is administered.

In another embodiment of the first or second aspect arfolitixorin is administered as a short IV infusion starting 45 minutes ± 2 minutes after the administration of oxaliplatin by infusion has commenced.

In a preferred embodiment of the first or second aspect arfolitixorin is administered as a short IV infusion over a period of 10 minutes ± 2 minutes.

In a preferred embodiment of the first or second aspect arfolitixorin is administered as a short IV infusion containing 200 mg/m2 over a period of 10 minutes ± 2 minutes.

In another preferred embodiment of the first or second aspect arfolitixorin is administered as a short IV infusion containing 300 mg/m2 over a period of 10 minutes ± 2 minutes. In another preferred embodiment of the first or second aspect arfolitixorin is administered as a short IV infusion containing 400 mg/m2 over a period of 10 minutes ± 2 minutes.

In another preferred embodiment of the first or second aspect arfolitixorin is administered as a short IV infusion containing 500 mg/m2 over a period of 12 minutes ± 2 minutes, such as 10 minutes ± 3 minutes.

In an embodiment of the first or second aspect the bolus administration of 5-FU in step d is replaced by an infusion of 5-FU or an administration of another fluoropyrimidine. In an embodiment of the first or second aspect the bolus administration of 5-FU in step d is omitted, and the short infusion of arfolitixorin is succeeded by a continuous IV infusion of 5-FU.

In an embodiment of the first or second aspect the short infusion of arfolitixorin in step c is succeeded directly by the continuous IV infusion of 5-FU of step e without the prior bolus administration of 5-FU in step d.

In an embodiment of the first or second aspect, the human patient has colorectal adenocarcinoma. In another embodiment of the first or second aspect, the human patient has not previously been treated for colorectal adenocarcinoma.

In an embodiment of any of the aspects of the invention, arfolitixorin is administered as the single diastereomer [6R]-5,10-methylene-tetrahydrofolate with a diastereomeric excess (d.e.) of >90% d.e., such as >93% d.e., such as >95% d.e., such as >98% d.e., such as >99% d.e., such as >99.5% d.e. or such as >99.9% d.e. In a preferred embodiment arfolitixorin is administered as a single diastereomer with a d.e. of >98% d.e.

In another embodiment of the invention, 5-fluorouracil is replaced by another fluorinated pyrimidine base such as capecitabine (Xeloda), ie. N4-pentyloxycarbonyl-5'-deoxy-5-fluoro-cytidine, tegafur, 5-fluoro-pyrimidinone, UFT, doxifluridine, 2'-deoxy-5-fluorouridine, 5'-deoxy-5-fluorouridine, 1-(2'-oxopropyl)-5-FU, and alkyl-carbonyl-5-FU, BOFA2, ftorafur (TS-1), and S-1.

In an embodiment arfolitixorin is employed in a solid form which is soluble in water, such as a lyophilisate or a salt, optionally stabilized by one or more suitable excipients and/or antioxidants such as citric acid or ascorbic acid or salt forms thereof.

In an embodiment of any of the aspects of the invention, a treatment cycle comprises two days. This regimen (ARFOX + bevazicumab) may be repeated every 14 days (+7 days) until disease progression (radiographic and/or clinical), unless unacceptable toxicity, death, patient withdrawal or general or specific changes in the patient's condition rendering the patient unacceptable for further treatment in the judgment of the Investigator.

In an embodiment of any of the aspects of the invention all steps a) - e) are thus repeated every 2 weeks until progressive disease (PD), or clear clinical deterioration according to the Investigator's judgment, and as long as the patient is tolerating the treatment and agrees to continue.

### EXAMPLE 1

A population pharmacokinetics (PK) model was developed to describe the PK characteristics of arfolitixorin, and to identify significant covariate-parameter relationships. The population PK analysis was based on data from a total of 118 subjects in five studies: ISO-FF-001 in healthy subjects, ISO-MC-091 in rectal cancer patients, ISO-CC-002 in colon cancer patients and ISO-CC-005 and ISO-CC-007 in patients with colorectal cancer. The covariate analysis included age, sex, race, indication, dose and co-medications. In studies ISO-CC-005 and ISO-CC-007, also aspartate aminotransferase, alanine aminotransferase, bilirubin, creatinine and creatinine clearance were evaluated.

The Pop-PK model was used to perform model based PK simulations using the software NON-MEM (version 7.4.3) to predict plasma concentrations of arfolitixorin after different IV doses and infusion length. A simulation was performed to enable comparison of different single dose regimens in patients with colorectal cancer and coadministration of 5-FU. 1000 subjects were simulated per regimen. Covariates were sampled (with replacement) from the popPKPD dataset to retain covariate correlations. As an example, Figure 5 is an overlay of the achieved exposure in ISO-CC-007 and ISO-FF-001 together with the model-predicted (simulated) plasma concentration following a short IV infusion of 200 mg/m² over 10 minutes.

### EXAMPLE 2

### Effectiveness of arfolitixorin vs. leucovorin in Thymidylate Synthase inhibition

The effectiveness of arfolitixorin vs. leucovorin in Thymidylate Synthase inhibition and in achieving high levels of [6R] 5,10-methylenetetrahydrofolate in metastatic tissue was investigated in the Modelle-001 Phase II clinical trial, link: Study Details | Modelle 001, TS-inhibition in Colorectal Liver Metastases Comparing Arfolitixorin and Calciumfolinate | ClinicalTrials.qov.

**Aim:** The Modelle-001 trial was designed to explore how a single intravenous bolus injection of ARF as compared to LV in the form of calcium folinate, together with 5-FU affects the inhibition of TS and, further, to investigate how different forms and doses of folates affect folate concentration and polyglutamylation in metastatic liver tissue.

**Patients and Methods:** Thirty adult patients with biopsy-verified colorectal cancer and liver metastases indicated for surgical removal were included at two Swedish University Hospitals. Both synchronous and metachronous liver metastases were accepted and most patients had received preoperative chemotherapy. The first six patients received a reduced 5-FU dosage (250 mg/m²), and an independent safety committee gave permission to escalate to full dosage. Twenty-four patients were randomized to receive 500 mg/m² 5-FU either in combination with 60 mg/m² LV (LV60, n = 12), 30 mg/m2 ARF (A30, n = 6) or 120 mg/m² ARF (A120, n = 6) as a bolus regimen (an i.v. injection for three minutes). Metastatic tissue was collected 60 minutes after folate administration, then 5-FU was given. After a 60-minute break, metastatic tissue was again collected. Folate levels and TS inhibition were determined by LC-MS/MS.

**Results:** The results showed that both A30 and A120 resulted in higher 5,10-methylenetetrahydrofolate (CH₂THF) concentrations in metastases, both before (p = 0.037 and p = 0.0003, respectively) and after (p = 0.031 and p = 0.0023, respectively) 5-FU treatment, as compared to LV60. Administration of A30 or A120 resulted in a higher ratio of poly/mono-glutamylated folates compared to LV60 after 5-FU administration (p = 0.045 and p = 0.0049, respectively). A dose-dependent TS inhibition was seen after administration of A30 (54.2% ± 189) vs A120 (83.8% ± 431) and both A30 and A120 seemed to give a better mean TS inhibition than LV60 (25.4% ± 301, p = 0.081 and p = 0.0026, respectively). The perioperative chemotherapy was well tolerated, and no Suspected Unexpected Serious Adverse Reaction occurred.

**Conclusions:** Significantly higher CH₂-THF concentration, ratio of poly-/mono-glutamylated CH2-THF and TS inhibition was found in colorectal liver metastases in patients receiving bolus injections of ARF compared to standard bolus LV therapy.

### EXAMPLE 3

### Concentration-dependent, additive cytotoxicity of ARF versus LV with 5-FU and oxaliplatin in colorectal cancer patient-derived tumoroids

As mentioned hereinabove, in the Phase 3 ISO-cc-007study the ARFOX regimen (arfolitixorin, 5-fluorouracil [5FU], oxaliplatin) + bevacizumab was unexpectedly comparable, but not superior, to standard mFOLFOX (leucovorin, 5FU, oxaliplatin) + bevacizumab in patients with advanced colorectal cancer (CRC).

A suboptimal dose hypothesis was supported by an initial screening study in CRC patient-derived tumoroids (PDTs), in which arfolitixorin displayed potent, concentration-dependent cytotoxicity in 5FU-treated PDTs, more so than leucovorin. In this example (Example 3) the screen was extended to investigate the combination of ARF vs. LV + oxaliplatin ± 5-FU.

Drug activity was assessed in 20 PDTs. Screening quality adhered to industry standards, with drug activity and cell viability measured using metabolic readouts and imaging. Statistical analysis included area-over-the-concentration-response-curve (AOC) and Wilcoxon paired-sample tests. The Bliss independence model was applied to determine whether combinations were synergistic, additive, or antagonistic.

5-FU and oxaliplatin showed heterogeneous responses across PDT models with median IC50 (half-maximal inhibitory concentration) values of 4.5 µM (range: 1.8->10) and 1.0 µM (range: 0.15-3.0), respectively. In a subset of 16 models, *KRAS* status (6/16 mutated, 38%) and Caudal Type Homeobox 2 (CDX2) protein expression was evaluated, but no relation with KRAS status and a trend with higher CDX2 protein expression was found for higher oxaliplatin sensitivity.

Expectedly, 20 µM arfolitixorin showed synergy with 5-FU, but not oxaliplatin. However, in contrast to leucovorin (at 8-80 µM), arfolitixorin showed single-agent activity with a median IC50 of 39 µM (range: 30-61), leading to an additional effect when combined with either 5FU or oxaliplatin. Surprisingly, there was no significant increase in activity when 20 µM leucovorin was added to 5FU-oxaliplatin (10:1), but there was a significant increase in activity with 20 µM arfolitixorin (13% higher AOC). Notably, for both 5FU and oxaliplatin, the modes of action at the tested pharmacological concentrations (up to 10 µM and 1 µM, respectively) were predominantly cytostatic, while arfolitixorin showed substantial cytotoxicity above 30 µM.The study demonstrates the potent, concentration-dependent cytotoxic effects of arfolitixorin, which increased the activity of 5FU-oxaliplatin in PDTs to a greater extent than with leucovorin. For arfolitixorin, both synergistic and additive effects were observed across combinations.

### EXAMPLE 4

ISO-cc-010 is a Phase 1b/2 study to determine the safety and preliminary efficacy of ARFOX + bevacizumab in patients with mCRC eligible for first-line therapy with a 5-FU, oxaliplatin, and bevacizumab regimen. Prior treatment with 5-FU, oxaliplatin or bevacizumab administration for mCRC or more than 6 cycles (3 months) of oxaliplatin exposure during adjuvant treatment, is prohibited.

The study will include a Phase 1b dose-finding part followed by a randomized Phase 2 dose optimization part.

All patients will receive treatment with ARFOX + bevacizumab every 14 days (+7 days) until progressive disease (PD), or clear clinical deterioration according to the Investigator's judgment, and as long as the patient is tolerating the treatment and agrees to continue.

During the Phase 1b part of the study, a dose escalation design will be utilized to identify the optimal dose and duration of administration of arfolitixorin to be used in combination with 5-FU, oxaliplatin and bevacizumab (Table 1). The planned dose escalation schedule will be to escalate the i.v. infusions from 120 mg/m² given as a 5 minutes i.v. short infusion up to 200 mg/m², 300 mg/m², 400 mg/m², and 500 mg/m², given as 10 minutes i.v. short infusions, until the MTD is reached. The dose escalation schema will be based on a Bayesian optimal interval (BOIN) design where the decision of dose escalation or de-escalation involves a simple comparison of the observed dose-limiting toxicities (DLTs) rate at the current dose with the prespecified dose escalation and de-escalation boundaries.

In **Phase 1b,** the dosing of arfolitixorin will begin at 120 mg/m² given as a 5-minute i.v. infusion. The **Phase 2** part of the study will be a randomized study where patients will be allocated to 1 of 2 dose levels of arfolitixorin, to be administered in combination with 5-FU, oxaliplatin and bevacizumab. In Phase 2, the dosing of arfolitixorin will be at the MTD as determined in Phase 1b or at a dose level below the MTD as determined in Phase 1b. In both phases, patients may receive treatment with ARFOX + bevacizumab every 14 days (+7 days) until PD, or clear clinical deterioration according to the Investigator's judgment, and as long as the patient is tolerating the treatment and agrees to continue.

**Table 1**

| | Bevacizumab | Oxaliplatin | Arfolitixorin* | 5-FU | 5-FU |
|---|---|---|---|---|---|
| **Cohort** | *At approx. -135 min (infusion 30-90 min)* | *At approx. -135 min (infusion 15-120 min)* | *At approx -25 min* | *At 0 min (bolus)* | *At approx. 3 min (infusion 46 hrs)* |
| Cohort 1 | 5 mg/kg | 85 mg/m² | 120 mg/m² | 400 mg/m² | 2400 mg/m² |
| Cohort 2 | 5 mg/kg | 85 mg/m² | 200 mg/m² | 400 mg/m² | 2400 mg/m² |
| Cohort 3 | 5 mg/kg | 85 mg/m² | 300 mg/m² | 400 mg/m² | 2400 mg/m² |
| Cohort 4 | 5 mg/kg | 85 mg/m² | 400 mg/m² | 400 mg/m² | 2400 mg/m² |
| Cohort 5 | 5 mg/kg | 85 mg/m² | 500 mg/m² | 400 mg/m² | 2400 mg/m² |

| | | | | | |
|---|---|---|---|---|---|
| ** In Cohort 1, ARF is administered as a 5-min infusion. In Cohorts 2-5, ARF is administered as a 10-min infusion* | | | | | |

### TREATMENT AND MATERIALS

**Arfolitixorin** ([6R]-5,10-methylene-tetrahydrofolic acid, [6R]-MTHF, CH2-THF, ARF) is formulated as a lyophylised powder containing 100 mg per vial (calculated as free acid). Dosing: Initial dose level of 120 mg/m² given as i.v. infusion over 5 minutes. Following doses given as i.v. infusion at a fixed dose over 10 minutes of 200, 300, 400 or 500 mg/m² until the MTD is reached. The regimen will be repeated every 2 weeks until PD or unacceptable toxicity.

**5-FU (5-fluorouracil)** is formulated as injection solution. The dosing of 5-FU will be as per label. 5-FU will be administered as a 400 mg/m² i.v. bolus (2-4 minutes) 15 minutes after completing the short infusion of ARF, followed by a continuous 2400 mg/m² i.v. infusion over 46 hours in each treatment cycle. The treatment will be repeated every 2 weeks until PD, premature discontinuation of ARF or unacceptable toxicity.

**Oxaliplatin** is formulated as a concentrated infusion solution. The dosing of oxaliplatin will be as per label. Oxaliplatin will be administered as an 85 mg/m² i.v. infusion on Day 1 in each treatment cycle and repeated every 2 weeks until PD, premature discontinuation of ARF or unacceptable toxicity.

**Bevacizumab** is formulated as a concentrated infusion solution.

The dosing of bevacizumab will be as per label. Bevacizumab will be administered as an i.v. infusion of 5 mg/kg of body weight given once every 2 weeks. Bevacizumab will be administered on Day 1 of every 2-week cycle and repeated every 2 weeks until PD, premature discontinuation of ARF or unacceptable toxicity.

## Claims

1. Arfolitixorin ([6R]-5,10-methylenetetrahydrofolate, [6R]-MTHF) for use in the treatment in a human patient of gastrointestinal cancer, including metastatic colorectal cancer, which treatment comprises the following steps:
a. administering a continuous IV infusion containing bevacizumab (5 mg/kg), followed by
b. administering a continuous IV infusion containing 85 mg/m² oxaliplatin, concomitant with
c. administering a short IV infusion over 10 min ± 3 min containing 200 - 500 mg/m² arfolitixorin, followed, 15 minutes after completing the short infusion, by
d. administering an IV bolus containing 400 mg/m² 5-fluorouracil (5-FU), followed immediately by
e. administering a continuous IV infusion containing 2400 mg/m² 5-FU over 46 hours,
wherein step b) and step c) are completed concurrently, and wherein step d) is initiated 25 minutes ± 3 min after initiating step c).

2. Arfolitixorin for use in a human patient according to claim 1 wherein all steps a) - e) are repeated every 2 weeks until progressive disease (PD), or clear clinical deterioration according to the Investigator's judgment, and as long as the patient is tolerating the treatment and agrees to continue.

3. Arfolitixorin for use in a human patient according to any one of claim 1-2, wherein the gastrointestinal cancer is a cancer form selected from colon cancer, colon adenocarcinoma and colorectal cancer (CRC) including metastatic CRC.

4. Arfolitixorin for use in a human patient according to any one of claim 1 - 3, wherein the bolus administration of 5-FU in step d is replaced by an infusion of 5-FU or an administration of another fluoropyrimidine.

5. Arfolitixorin for use in a human patient according to any one of claim 1 - 3, wherein step d is not performed, but where the short infusion of arfolitixorin in step c is succeeded by a continuous IV infusion of 5-FU.

6. Arfolitixorin for use in a human patient according to any one of the preceding claims wherein genotype testing has been performed of said tumor by dPCR analysis of tumor tissue.

7. Arfolitixorin for use in a human patient according to any one of the preceding claims wherein genotype testing has been performed of said tumor by circulating biomarker analysis on an isolated blood sample from said human patient.

8. Arfolitixorin for use in a human patient according to any one of claim 1 - 7, wherein said patient is previously untreated for gastrointestinal cancer.

9. Arfolitixorin for use in a human patient according to any one of the preceding claims wherein arfolitixorin is employed as a solid form which is soluble in water, such as a lyophilizate or a salt, optionally stabilized by one or more suitable excipients and/or antioxidants such as citric acid or ascorbic acid or salt forms thereof.

10. Arfolitixorin for use in a human patient according to any one of the preceding claims wherein 5-fluorouracil (5-FU) is replaced by another fluoropyrimidine such as capecitabine (Xeloda), i.e. N4-pentyloxycarbonyl-5'-deoxy-5-fluorocytidine, tegafur, 5-fluoro-pyrimidinone, UFT, doxifluridine, 2'-deoxy-5-fluorouridine, 5'-deoxy-5-fluoro-uridine, 1-(2'-oxopropyl)-5-FU, BOF-A2, ftorafur(TS-1), and S-1.

11. Arfolitixorin for use in a human patient according to any one of the preceding claims wherein 5-FU is replaced by another fluoropyrimidine selected from 2'-deoxy-5 fluoro-uridine and 5'-deoxy-5-fluorouridine.
